# EUROPEAN PATENT SPECIFICATION

(11) **EP 4 410 781 B1**
(45) Date of publication and mention of the grant of the patent: **23.09.2026**
(21) Application number: 22885743.9
(22) Date of filing: 18.10.2022
(51) Int. Cl.: C07D 319/12, C08G 63/08, C08G 63/88

(54) **METHOD AND SYSTEM FOR CONTINUOUSLY PREPARING LACTIDE BY STEP CONTROL**
VERFAHREN UND SYSTEM ZUR KONTINUIERLICHEN HERSTELLUNG VON LACTID DURCH SCHRITTSTEUERUNG
PROCÉDÉ ET SYSTÈME DE PRÉPARATION EN CONTINU DE LACTIDE PAR CONTRÔLE D'ÉTAPES

(30) Priority: 31.10.2021 CN 202111279100
(43) Date of publication of application: 07.08.2024
(73) Proprietor: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: SUN, Qimei, Dalian, Liaoning 116045 (CN); ZHOU, Feng, Dalian, Liaoning 116045 (CN); LI, Lanpeng, Dalian, Liaoning 116045 (CN); LIU, Laiwu, Dalian, Liaoning 116045 (CN); BAI, Fudong, Dalian, Liaoning 116045 (CN); ZHANG, Lei, Dalian, Liaoning 116045 (CN); BAI, Yuli, Dalian, Liaoning 116045 (CN); WANG, Pengxiang, Dalian, Liaoning 116045 (CN); LI, Xiuzheng, Dalian, Liaoning 116045 (CN)
(74) Representative: karo IP
(86) International application number: PCT/CN2022/125901
(87) International publication number: WO 2023/071865

(56) References cited:
- WO-A1-2014/000277
- WO-A1-2015/080402
- CN-A- 1 446 209
- CN-A- 101 434 594
- CN-A- 111 153 886
- CN-A- 111 548 339
- CN-A- 112 480 063
- CN-A- 112 898 266
- JP-A- 2009 298 935
- US-A1- 2021 188 799

## Description

### CROSS-REFERENCE TO RELATED APPLICATIONS

The application claims the benefit of the Chinese patent application No. "202111279100.9", filed on October 31, 2021.

### TECHNICAL FIELD

The present invention belongs to the technical field of biodegradable materials and particularly relates to a method and a system for continuously preparing lactide by step control.

### BACKGROUND ART

The global consumption of disposable plastic products is up to 120 million tons every year at present, among them, only 10% is recycled, the other about 12% is incinerated, and over 70% is discarded into the soil, air, and ocean. The amount of plastic garbage thrown into the ocean is over 8 million tons every year, and the figure is continuously rising, thus the amount of plastic garbage in the ocean in the world is estimated to reach 250 million tons by 2025. Traditional disposable plastic products have short service life, but have stable physical and chemical properties and are hard to naturally degradable; in addition, a large amount of disposable plastic product wastes have caused various environmental problems, which have seriously impaired the land and water body as well as health and safety of animals and human beings. The relevant policies or regulations for controlling or banning disposable non-degradable plastic articles have been introduced in nearly 90 countries and regions around the world.

The currently commercialized biodegradable plastics comprise polylactic acid (PLA), polyhydroxyalkanoate (PHA), polybutylene succinate (PBS), polybutylene succinate adipate (PBSA), polybutylene adipate terephthalate (PBAT) and the like, among them, PLA is the most widely applied at present and has the most prominent application prospect. It not only has the basic performance of common high molecular materials, but also has superior processability, physical and mechanical performance, and biodegradability, it can be widely applied in the packaging industry, textile industry, agricultural industry, consumer goods market, and the like, thus PLA has gradually developed into the basic bulk raw material necessary for the national economy and social development in China.

Typically, the polylactic acid (PLA) is synthesized using a two-step process. The specific method for synthesizing PLA with a two-step process comprises the following steps: Step 1, preparing lactide from lactic acid; and Step 2, subjecting the lactide to a ring-opening polymerization to prepare the polylactic acid, and the molecular weight of PLA obtained in the process may reach 100,000 to 1,000,000. The lactide is the key to the whole synthesis process, the process barrier is relatively high, and the lactide is usually prepared through polycondensation and depolymerization processes in the presence of a catalyst and under the high-temperature and high-vacuum system, and the process is prone to cause racemization of lactide. The key reason maybe that the depolymerization process for generating lactide mainly takes place in the catalytic reaction of "back-biting" ester on the molecular chain of the lactic acid oligomer, and the specific process is as follows: under the effects of a catalyst, high temperature, and high vacuum, the carbonyl on a lactic acid oligomer chain is activated, and the hydroxyl at the first segment of the chain attacks the positively charged carbonyl so that an ester bond is ruptured ("positive biting" process) to form an L/D-lactide; however, in the presence of a basic oxide, an excessive amount of catalyst or at an excessively high temperature, the carboxylic acid anion at the end of the lactic acid oligomer attacks the chiral carbon atom on the unit adjacent to the lactic acid unit, such that the bond between the methine carbon and the ester oxygen bond is broken ("back-biting" process), and the configuration is reversed to obtain meso lactide (m-lactide), as shown in formula (1). The existence of m-lactide, on one hand, will influence the optical purity of lactide, thereby affecting the ring-opening polymerization process of lactide, so that the prepared PLA has low molecular weight; on the other hand, it will destroy the regularity of the PLA structure, such that the crystallinity and the mechanical property of the PLA are reduced.

Therefore, the crude lactide produced through depolymerization reaction needs to be purified and refined by processes such as solvent recrystallization, water extraction, rectification, and melt crystallization to reduce the content of m-lactide in the product; however, because the physical and chemical properties of L-lactide and *m-*lactide are similar, and the lactide has the characteristics of high condensation point, boiling point, heat sensitivity, the separation process is difficult, the overall yield is only about 40%-60%, and the overall economical efficiency is low. Therefore, racemization in the synthesis process of lactide is a key factor influencing the quality and yield of the lactide, it is also the key and difficult point of the technical research of the lactide at home and abroad at present.

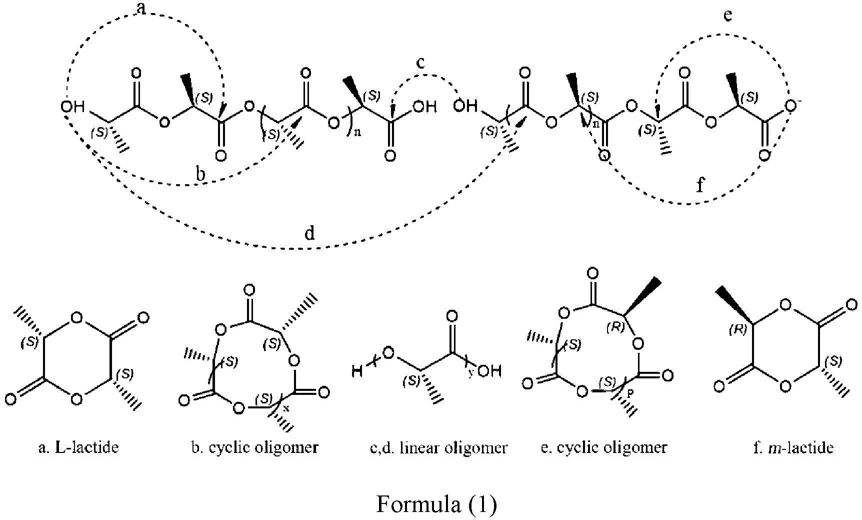

Morteza Ehsani, et al. have studied in detail the influence of temperature, catalyst and other factor on the depolymerization process in the article entitled "Lactide synthesis optimization: investigation of the temperature, catalyst and pressure effects", it is found in the study of reaction temperature, when the reaction temperature is low, m-Lactide is generated in a small amount, the content of m-Lactide is obviously increased along with the temperature rise, and when the reaction temperature is 230°C, the content of m-Lactide reaches 25.52%; when the influence of stannous oxide, stannous chloride, stannous octoate, antimony trioxide and sulfuric acid on the synthesis process of lactide is inspected, the purity of the lactide product prepared by using SnCl₂ and sulfuric acid as catalysts is highest and the content of m-lactide is the least; however, the catalyst concentration should not be too high, and taking SnCl₂ as an example, the yield of lactide is increased with the increase of SnCl₂ concentration, but an excessive amount of catalyst will accelerate the racemization reaction rate.

US5502215A discloses a method for refining and purifying lactide, the patent application uses SnO as a catalyst, and a lactic acid oligomer is added into a kettle-type three-neck flask to carry out depolymerization reaction to prepare a crude D,L-lactide, the process needs strong stirring and the reaction temperature is high (the reaction temperature is 220°C), the obtained product has low purity, the coking and carbonization of a substrate at the kettle bottom are serious, and the racemization of the lactide is also intensified by the high-temperature reaction.

US6326458B discloses a continuous process for the manufacture of lactide and lactide polymer, which adopts a falling film-type column tube evaporator in a depolymerization reactor in a depolymerization section for the preparation of lactide, wherein the lactic acid oligomer enters the top of the evaporator, lactide steam is extracted from the bottom of the column tube evaporator, and the unreacted lactic acid oligomer is discharged from a lower discharge port. The reaction temperature required in the falling film reaction process is relatively low, and the racemization probability of lactide in the depolymerization process can be effectively reduced, but the lactide yield is low, it is generally required to reduce the feeding rate for maintaining the high lactide yield, but the retention time of oligomers on the surface of said falling film reactor is increased, the non-depolymerized lactic acid oligomer is rapidly polymerized in a high-temperature high-vacuum system, the molecular weight of the oligomers is large, the depolymerization rate is further influenced, and it is prone to cause coking and carbonization of the oligomers on the surface of the falling film column tube reactor.

CN111153886A discloses a synthesis method and device for rapidly producing lactide at high yield, the method comprises the following steps: adding a lactic acid single component or lactic acid into a catalyst double component, enabling the mixture to enter an oligomer preparation system through a mixer, increasing the residence time through bottom circulation, synthesizing oligomeric lactic acid, and enabling a gas-phase component to pass through a rectification system, so as to improve the yield of oligomeric lactic acid; removing unreacted lactic acid and water from the oligomeric lactic acid through a purification device; and adding a catalyst into the light-component-removed oligomeric lactic acid, allowing the mixture to pass through a mixer, allowing the mixture to enter a depolymerization reactor, depolymerizing so as to obtain lactide, allowing heavy components to enter the depolymerization reactor again through reflux, and allowing light components to pass through a purification and recovery system in order to obtain the lactide product. With the adoption of the device, lactide can be efficiently synthesized, crude lactide with a yield of 94%-98% can be obtained within a short residence time of 0.5-5 minutes, the molecular weight of the heavy-component polylactic acid is slowly increased, and the heavy-component polylactic acid can be returned for depolymerization; after the light components pass through a simple purification system, the content of L-lactide, D-lactide or D,L-lactide in the lactide product is 94%-98%, and the content of meso-lactide is 0.5%-5.5%. However, the depolymerized heavy component in the invention directly flows back into the depolymerization reactor, which not only affects the stability of the depolymerization reaction, but also easily increases the coking and carbonization probability of reaction substrates on the surface of the reactor along with the increase of molecular weight of the heavy component and the accumulation of said catalyst, increases the racemization degree of lactide, and affects the continuous and stable operation of the reaction. JP2009298935A discloses synthesis method and device of lactide and polylactic acid. The problem of this invention is to synthesize lactide with less formation of optical isomers. In this invention, concentrated lactic acid is supplied to a lactic acid condensation device to produce a lactic acid oligomer. This oligomer is supplied to a tower-type first continuous depolymerization device, where it is brought into contact and mixed with a catalyst to generate lactide. The residue of the reaction solution is continuously supplied to a second continuous depolymerization device to which a centrifugal thin-film evaporator is applied, for further lactide production. The residual liquid is then supplied to a third continuous depolymerization device to which a horizontal twin-screw stirrer is applied, for additional lactide generation. The lactide produced in each depolymerization device is processed by a partial condenser or the like to condense impurities, which are then refluxed to the lactic acid condensation device. The lactide vapor is condensed and recovered by a lactide condenser or the like, and each portion is processed by a purification device to produce the final lactide. CN111548339A discloses a process for preparing glycolide from glycolate, which belongs to the technical field of chemical products. The process comprises the following steps: the reaction raw material (glycolate) and a catalyst undergo a preliminary reaction in a prepolymerization kettle; the resulting mixture is transferred to a final polymerization kettle connected to the prepolymerization kettle; the oligomer formed by the reaction in the final polymerization kettle flows into a depolymerization kettle for a depolymerization reaction; part of the crude glycolide obtained in the depolymerization kettle directly flows into a crude product storage tank, while the remaining depolymerized materials are sent into a wiped film evaporator by a circulation pump; in the wiped film evaporator, crude glycolide and other materials are separated, with the crude glycolide collected in the crude product storage tank and the other materials reflowing into the depolymerization kettle to continue the depolymerization reaction; finally, the crude glycolide collected in the crude product storage tank is purified by a melt crystallizer to obtain pure glycolide. The polymerization process designed in this invention is divided into two steps, which effectively reduces the energy consumption in production; meanwhile, the use of the wiped film evaporator avoids side reactions such as further polymerization and coking caused by the excessively long residence time of the oligomer in the depolymerization kettle.

### SUMMARY OF THE INVENTION

The present invention aims to overcome the defects in the prior art and provides a method and a system for continuously preparing lactide by step control. The invention performs the step control on the multi-stage cascade depolymerization, realizes the high-efficiency depolymerization of the lactic acid oligomer, reduces racemization degree of lactide and the coking carbonization probability of the substrate, ensures continuous stable operation of the depolymerization process and stability of the composition of the crude lactide product, and improves the depolymerization reaction rate and production efficiency as well as the yield of lactide.

The invention provides a method for continuously preparing lactide by step control, the method comprises the following steps:
(1) reacting a lactic acid oligomer and a depolymerization catalyst in a first depolymerization reaction unit to obtain a first liquid-phase material;
(2) circulating the first liquid-phase material in a second depolymerization reaction unit for reaction until the molecular weight of the liquid-phase material is higher than 6000 to obtain a second liquid-phase material;
(3) circulating the second liquid-phase material in a third depolymerization reaction unit for reaction until the molecular weight of the liquid-phase material is higher than 10,000;
(4) collecting gas-phase crude lactide from the first depolymerization reaction unit, the second depolymerization reaction unit, and the third depolymerization reaction unit, and then purifying the same;
   the second depolymerization reaction unit comprises at least one second depolymerization reactor and at least one second circulation tank, the first liquid-phase material and an optional protonated solvent carry out the reaction in the second depolymerization reactor, the reacted liquid-phase material enters the second circulation tank; when the molecular weight of the liquid-phase material is 6,000 or less, the liquid-phase material in the second circulation tank is recycled to the second depolymerization reactor for further reaction; when the molecular weight of the liquid-phase material is larger than 6,000, the liquid-phase material in the second circulation tank is conveyed to the third depolymerization reaction unit for reaction;
   the third depolymerization reaction unit comprises at least one third depolymerization reactor and at least one third circulation tank, the second liquid-phase material carries out the reaction in the third depolymerization reactor, the reacted liquid-phase material enters the third circulation tank; when the molecular weight of the liquid-phase material is 10,000 or less, the liquid-phase material in the third circulation tank is recycled to the third depolymerization reactor for further reaction; when the molecular weight of the liquid-phase material is larger than 10,000, the liquid-phase material in the third circulation tank is discharged.

Preferably, the first depolymerization reaction unit comprises a first depolymerization reactor and a first circulation tank, the lactic acid oligomer and the depolymerization catalyst carry out the reaction in the first depolymerization reactor, and the first liquid-phase material obtained after the reaction enters the first circulation tank.

Preferably, the reaction of step (2) is performed in the presence of a protonated solvent.

The present invention also provides a system for continuously preparing lactide by step control, the system comprises:
a first depolymerization reaction unit, wherein a lactic acid oligomer and a polymerization catalyst carry out the reaction in the first depolymerization reaction unit;
a second depolymerization reaction unit, wherein a liquid-phase material from the first depolymerization reaction unit and an optional protonated solvent circulate in the second depolymerization reaction unit and carry out the reaction until the molecular weight of the liquid-phase material is higher than 6,000;
a third depolymerization reaction unit, wherein the liquid-phase material with a molecular weight higher than 6,000 from the second depolymerization reaction unit circulates in the third depolymerization reaction unit and carries out the reaction until the molecular weight of the liquid-phase material is higher than 10,000; and
a device for collecting and purifying the gas-phase crude lactide from the first depolymerization reaction unit, the second depolymerization reaction unit, and the third depolymerization reaction unit;
the second depolymerization reaction unit comprises at least one second depolymerization reactor and at least one second circulation tank, the first liquid-phase material and an optional protonated solvent carry out the reaction in the second depolymerization reactor, the reacted liquid-phase material enters the second circulation tank; when the molecular weight of the liquid-phase material is 6,000 or less, the liquid-phase material in the second circulation tank is recycled to the second depolymerization reactor for further reaction; when the molecular weight of the liquid-phase material is larger than 6,000, the liquid-phase material in the second circulation tank is conveyed to the third depolymerization reaction unit for reaction;
the third depolymerization reaction unit comprises at least one third depolymerization reactor and at least one third circulation tank, the second liquid-phase material carries out the reaction in the third depolymerization reactor, the reacted liquid-phase material enters the third circulation tank; when the molecular weight of the liquid-phase material is 10,000 or less, the liquid-phase material in the third circulation tank is recycled to the third depolymerization reactor for further reaction; when the molecular weight of the liquid-phase material is larger than 10,000, the liquid-phase material in the third circulation tank is discharged.

Compared with the prior art, the present invention has the beneficial effects as follows:
(1) the invention adopts the multi-stage cascade depolymerization reaction and carries out the step control on the reaction, each stage of the depolymerization reaction unit mainly comprises a depolymerization reactor and a circulation tank, and performs regulation and control according to the conditions of the molecular weight of lactic acid oligomer in the circulation tank, thereby realizing the high-efficiency depolymerization of the lactic acid oligomer, reducing the racemization degree of lactide and coking carbonization probability of the substrate, and ensuring the conversion rate of the lactic acid oligomer and the system stability throughout the whole reaction process. The conversion rate of the lactic acid oligomer in the whole process may reach 97.0% or more.
(2) Different operating parameters (including reaction temperature, absolute pressure (also called vacuum degree) and reaction time or one-way reaction time, and the like) are arranged for the different cascade depolymerization reactors, so that the feeding stability of lactic acid oligomer in each level of depolymerization system and the stable control of reaction operating parameters are ensured, and the reaction rate of each cascade depolymerization reactor is increased, thereby improving the whole reaction efficiency, ensuring the stability of the discharge quality and yield of the crude lactide, and realizing the continuous and stable operation of the whole depolymerization process. Compared with a single circulation depolymerization system process, the depolymerization efficiency of the cascade depolymerization process is improved by 30%.
(3) In a preferred embodiment, based on step control on the multi-stage series depolymerization reaction unit, and in combination with the use of a protonated solvent, the racemization degree of lactide and the coking carbonization probability of the substrate in the lactide synthesis process can be further reduced, and the product quality is improved. The m-lactide content in the obtained crude lactide of the three depolymerization reaction units can be controlled within 6.0%. Compared with a kettle-type cyclic depolymerization reactor, the racemization degree of lactide is reduced by more than 50%, and compared with the one-way wiped film evaporator or a multi-depolymerization reactor which is connected in series with a non-step control, the conversion rate of lactic acid oligomer is improved by more than 10%.

### BRIEF DESCRIPTION OF THE DRAWINGS

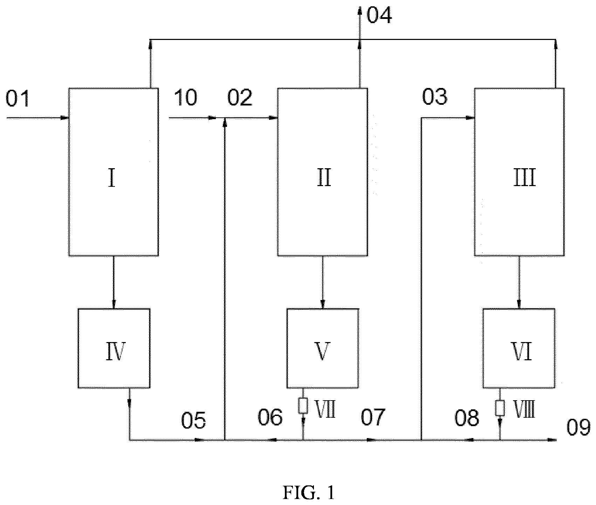

FIG. 1 is a structural schematic diagram of one specific embodiment of the system for continuously preparing lactide by step control according to the present invention.

### DESCRIPTION OF REFERENCE SIGNS

I - First depolymerization reactor
II - Second depolymerization reactor
III - Third depolymerization reactor
IV - First circulation tank
V - Second circulation tank
VI - Third circulation tank
VII -First molecular weight detection and control assembly
VIII - Second molecular weight detection and control assembly
01 - Lactic acid oligomer
02 - Second circulation tank feedstock
03 - Third circulation tank feedstock
04 - Crude lactide
05 - First circulation tank discharge
06 - Second circulation tank recycled material
07 - Second circulation tank discharge
08 - Third circulation tank recycled material
09 - Lactic acid high polymer
10 -Protonated solvent

### DESCRIPTION OF THE PREFERRED EMBODIMENT

The preferred embodiments of the present invention will be described in detail below with reference to the appended drawings. It should be understood that the specific embodiments described herein merely serve to illustrate or explain the invention, instead of imposing limitation thereto.

The invention provides a method for continuously preparing lactide by step control, the method comprises the following steps:
(1) reacting a lactic acid oligomer and a depolymerization catalyst in a first depolymerization reaction unit to obtain a first liquid-phase material;
(2) circulating the first liquid-phase material in a second depolymerization reaction unit for reaction until the molecular weight of the liquid-phase material is higher than 6000 to obtain a second liquid-phase material;
(3) circulating the second liquid-phase material in a third depolymerization reaction unit for reaction until the molecular weight of the liquid-phase material is higher than 10,000;
(4) collecting gas-phase crude lactide from the first depolymerization reaction unit, the second depolymerization reaction unit, and the third depolymerization reaction unit, and then purifying the same.

In the method of the invention, step (1) is to carry out a preliminary reaction, realize continuous and stable reaction of materials by controlling conditions and achieve the stability of discharging; step (2) serves to allow further reaction of the materials, and racemization of the product is controlled by regulating and controlling the single-pass reaction time so that the reaction efficiency and conversion rate are further improved; step (3) relates to the deep reaction of materials, enabling materials with larger molecular weight to further participate in depolymerization reaction by adjusting the temperature and the vacuum degree, and controlling racemization of the product by adjusting and controlling the single-pass reaction time of the materials, so that the product meets the product requirements, and ensuring the conversion rate of reaction. The multi-stage reactions (preferably three-stage reactions) in steps (1) to (3) are mutually cooperated and associated, thereby ensuring the stability of the discharge quality and yield of the crude lactide, and realizing the continuous and stable operation of the whole depolymerization process.

In the method of the present invention, the lactic acid oligomer in step (1) may have a molecular weight within a range of 800-3,000, preferably within a range of 1,200-2,800. The molecular weight of the lactic acid oligomer herein refers to a weight-average molecular weight.

In the present invention, the method may further comprise preparing the lactic acid oligomer according to the following process: sequentially dehydrating and polycondensing the L-lactic acid and/or D-lactic acid. The dehydration procedure is mainly used for removing free water in the lactic acid and can adopt a normal pressure or reduced pressure mode. The polycondensation conditions may comprise the reaction temperature within a range of 140-170°C, the absolute pressure within a range of 1,000-2,000Pa, and the reaction time within a range of 0.5-4 h.

In the method of the present invention, the polymerization catalyst in step (1) is preferably used in an amount of 0.4%-3%, more preferably 0.8%-2% by mass of the lactic acid oligomer.

In the method of the present invention, the polymerization catalyst in step (1) is preferably tin-based catalyst, more preferably at least one of stannous octoate, SnCl₂, and SnO.

In the method of the present invention, the reaction conditions in step (1) preferably comprise the reaction temperature within a range of 180-200°C, the absolute pressure within a range of 500-1,500Pa, and the reaction time within a range of 3-8 min.

According to a specific embodiment of the present invention, the first depolymerization reaction unit comprises a first depolymerization reactor and a first circulation tank, the lactic acid oligomer and the depolymerization catalyst carry out the reaction in the first depolymerization reactor, and the first liquid-phase material obtained after the reaction enters the first circulation tank.

In a case of preferably, the liquid level in the first circulation tank is maintained within the range of 50%-70%, the pressure is kept within the range from 10 kPa to atmospheric pressure, and the temperature is maintained within the range of 160-200°C. In this case of preferably, the possibility of further performing intermolecular polymerization of the lactic acid oligomer can be reduced, and the coking and carbonization may be alleviated.

In the method of the present invention, preferably, the conversion rate of the lactic acid oligomer in the reaction of step (1) is controlled to be between 50% and 60% by controlling the amount of the lactic acid oligomer conveyed to the first depolymerization reactor. In this case of preferably, the possibility of further performing intermolecular polymerization of the lactic acid oligomer can be reduced, and the coking and carbonization may be alleviated.

In the method of the present invention, preferably, the reaction of step (2) is performed in the presence of a protonated solvent. In a specific embodiment, a first liquid-phase material from the first depolymerization reaction unit is mixed with a protonated solvent and the mixture is delivered to the second depolymerization reaction unit. In this case of preferably, the use of a protonated solvent can further reduce the racemization degree of lactide during the synthesis process and the coking carbonization probability of the substrate, and can improve the product quality.

In the present invention, the protonated solvent may be at least one of a diamine having 12 or more carbon atoms, and a diol having 12 or more carbon atoms. Preferably, the melting temperature of the protonated solvent is within a range of 80-160°C, more preferably within a range of 100-160°C. Further preferably, the protonated solvent is at least one of C12-C18 diamine and C12-C18 diol. More preferably, the protonated solvent is at least one of dodecanediamine, tetradecanediamine, hexadecanediamine, tetradecanediol, and hexadecanediol.

In the method of the invention, the protonated solvent may be used in an amount of 0.1%-6%, preferably 1%-3% by mass of the lactic acid oligomer in the reaction of step (2).

According to a specific embodiment of the present invention, the second depolymerization reaction unit comprises at least one second depolymerization reactor and at least one second circulation tank, the first liquid-phase material and an optional protonated solvent carry out the reaction in the second depolymerization reactor, the reacted liquid-phase material enters the second circulation tank; when the molecular weight of the liquid-phase material is 6,000 or less, the liquid-phase material in the second circulation tank is recycled to the second depolymerization reactor for further reaction; when the molecular weight of the liquid-phase material is larger than 6,000, the liquid-phase material in the second circulation tank is conveyed to the third depolymerization reaction unit for reaction. Preferably, when the molecular weight of the liquid-phase material is within a range of 3,000-6,000, the liquid-phase material in the second circulation tank is recycled to the second depolymerization reactor for further reaction; when the molecular weight of the liquid-phase material is more than 6,000 and less than 10,000, the liquid-phase material in the second circulation tank is conveyed to the third depolymerization reaction unit for reaction.

In the specific embodiment mentioned above, the second depolymerization reaction unit may comprise one second depolymerization reactor and one second circulation tank, or may comprise two or more second depolymerization reactors and two or more second circulation tanks. Preferably, when the second depolymerization reaction unit comprises two or more second depolymerization reactors and two or more second circulation tanks, each second depolymerization reactor is respectively and accordingly configured with a second circulation tank, and each second depolymerization reactor and its corresponding second circulation tank constitute a cyclic reaction unit. In a specific example, the second depolymerization reaction unit comprises two second depolymerization reactors and two second circulation tanks, wherein the second depolymerization reactor (A) and the second circulation tank (a) constitute one circulation reaction unit (2-1), the second depolymerization reactor (B) and the second circulation reactor (b) constitute another circulation reaction unit (2-2), the liquid-phase material from the first depolymerization reaction unit (i.e., the first liquid-phase material) initially enters the second depolymerization reactor (A) of circulation reaction unit (2-1) and carries out reaction, the reacted liquid-phase material enters the second circulation tank (a), when the molecular weight of the liquid-phase material is 4,500 or less (e.g., within the range of 3,000-4,500), the liquid-phase material in the second circulation tank (a) is recycled to the second depolymerization reactor (A) for further reaction; when the molecular weight of the liquid-phase material is larger than 4,500 and less than 6,000, the liquid-phase material in the second circulation tank (a) is conveyed to the second depolymerization reactor (B) of the circulation reaction unit (2-2), the reacted liquid-phase material enters the second circulation tank (b). When the molecular weight of the liquid-phase material is 6,000 or less (e.g., within the range of 4,500-6,000), the liquid-phase material in the second circulation tank (b) is recycled to the second depolymerization reactor (B) for further reaction; when the molecular weight of the liquid-phase material is larger than 6,000 and less than 10,000, the liquid-phase material in the second circulation tank (b) is conveyed to the third depolymerization reaction unit for reaction. In the actual operation process, the larger the number of cyclic reaction units consisting of one depolymerization reactor and one circulation tank included in the second depolymerization reaction unit, the finer control of the molecular weight of different stages of the lactic acid oligomer can be achieved, thereby producing better reaction effect, however, the second depolymerization reaction unit preferably comprises one second depolymerization reactor and one second circulation tank by comprehensively considering both the costs and the magnitude of effect improvement.

In the method of the present invention, the reaction conditions in the second depolymerization reactor may comprise the reaction temperature within a range of 200-220°C, the absolute pressure within a range of 400-1,000Pa, and the one-way reaction time within a range of 2-5 min.

In the method of the present invention, preferably, the feeding amount of the lactic acid oligomer in a one-way reaction of the second depolymerization reactor is 3-5 times the actual reaction amount. In this case of preferably, the residence time of the lactic acid oligomer on the surface of the second depolymerization reactor can be reduced, thereby inhibiting the occurrence of the polymerization reaction, improving the yield, and ensuring the product quality.

In the method of the present invention, it is preferable that the liquid level in the second circulation tank is maintained within the range of 50%-70%, the pressure is kept within the range from 10 kPa to atmospheric pressure, and the temperature is maintained within the range of 160-200°C.

In the method of the present invention, the conversion rate of lactic acid oligomer in the depolymerization reaction process of step (2) can reach 70% or more.

According to a specific embodiment of the present invention, the third depolymerization reaction unit comprises at least one third depolymerization reactor and at least one third circulation tank, the second liquid-phase material carries out the reaction in the third depolymerization reactor, the reacted liquid-phase material enters the third circulation tank; when the molecular weight of the liquid-phase material is 10,000 or less, the liquid-phase material in the third circulation tank is recycled to the third depolymerization reactor for further reaction; when the molecular weight of the liquid-phase material is larger than 10,000, the liquid-phase material in the third circulation tank is discharged.

In the specific embodiment mentioned above, the third depolymerization reaction unit may comprise one third depolymerization reactor and one third circulation tank, or comprise two or more third depolymerization reactors and two or more third circulation tanks. Preferably, when the third depolymerization reaction unit comprises two or more third depolymerization reactors and two or more third circulation tanks, each third depolymerization reactor is respectively and accordingly configured with a third circulation tank, and each third depolymerization reactor and its corresponding third circulation tank constitute a cyclic reaction unit. In a specific example, the third depolymerization reaction unit comprises two third depolymerization reactors and two third circulation tanks, wherein the third depolymerization reactor (C) and the third circulation tank (c) constitute one circulation reaction unit (3-1), the third depolymerization reactor (D) and the third circulation reactor (d) constitute another circulation reaction unit (3-2), the liquid-phase material from the second depolymerization reaction unit (i.e., the second liquid-phase material) initially enters the third depolymerization reactor (C) of circulation reaction unit (3-1) and carries out reaction, the reacted liquid-phase material enters the third circulation tank (c), when the molecular weight of the liquid-phase material is 8,000 or less (e.g., larger than 6,000 and less than or equal to 8,000), the liquid-phase material in the third circulation tank (c) is recycled to the second depolymerization reactor (C) for further reaction; when the molecular weight of the liquid-phase material is larger than 8,000 and less than 10,000, the liquid-phase material in the third circulation tank (c) is conveyed to the third depolymerization reactor (D) of the circulation reaction unit (3-2), the reacted liquid-phase material enters the third circulation tank (d). When the molecular weight of the liquid-phase material is 10,000 or less, the liquid-phase material in the third circulation tank (d) is recycled to the third depolymerization reactor (D) for further reaction; when the molecular weight of the liquid-phase material is larger than 10,000, the liquid-phase material in the third circulation tank (d) is discharged out of the system. In the actual operation process, the larger the number of cyclic reaction units consisting of one depolymerization reactor and one circulation tank included in the third depolymerization reaction unit, the finer control of the molecular weight of different stages of the lactic acid oligomer can be achieved, thereby producing better reaction effect, however, the third depolymerization reaction unit preferably comprises one third depolymerization reactor and one third circulation tank by comprehensively considering both the costs and the magnitude of effect improvement.

In the method of the present invention, the reaction conditions in the third depolymerization reactor may comprise the reaction temperature within a range of 220-240°C, the absolute pressure within a range of 200-800Pa, and the one-way reaction time within a range of 1-4 min.

In the method of the present invention, preferably, the feeding amount of the lactic acid oligomer in the one-way reaction of the third depolymerization reactor is 4-6 times the actual reaction amount. In this case of preferably, the residence time of the lactic acid oligomer on the surface of the third depolymerization reactor can be reduced, thereby inhibiting the occurrence of the polymerization reaction, improving the yield, and ensuring the product quality.

In the method of the present invention, preferably, the liquid level in the third circulation tank is maintained within the range of 10%-30%, the pressure is kept within the range from 10 kPa to atmospheric pressure, and the temperature is maintained within the range of 160-200°C.

In the method of the invention, the liquid-phase material having a molecular weight of more than 10,000 discharged from the third depolymerization reaction unit is a lactic acid high polymer, and the lactic acid can be recycled by hydrolysis.

In the method of the present invention, the conversion rate of lactic acid oligomer in the depolymerization reaction process of step (3) can reach 70% or more.

According to the method of the invention, the conversion rate of the lactic acid oligomer in the whole step control depolymerization reaction process can reach 97.0% or more.

In the method of the present invention, preferably, the reactors in the first depolymerization reaction unit, the second depolymerization reaction unit, and the third depolymerization reaction unit (i.e., the first depolymerization reactor, the second depolymerization reactor, and the third depolymerization reactor) are each a wiped film depolymerization reactor, more preferably a thin film evaporator, a molecular distillation evaporator, or other stirred film evaporator.

According to the method of the invention, the gas-phase crude lactide generated by three depolymerization reaction units is discharged from the top of each depolymerization reactor, and the crude lactide is composed of the following ingredients by mass: 82%-92% of L-lactide, 1.0%-6% of m-lactide, 0.5%-6% of L-lactic acid, and 1.5%-6% of dimer and trimer.

According to the method of the invention, gas-phase crude lactide generated by three depolymerization reaction units enters a separation and purification process, and can be directly refined by rectification or other purification and refinement processes, and the quality of the obtained product meets the requirements of a polymer grade lactide monomer.

The invention provides a system for continuously preparing lactide by step control, the system comprises:
a first depolymerization reaction unit, wherein a lactic acid oligomer and a polymerization catalyst carry out the reaction in the first depolymerization reaction unit;
a second depolymerization reaction unit, wherein a liquid-phase material from the first depolymerization reaction unit and an optional protonated solvent circulate in the second depolymerization reaction unit and carry out the reaction, until the molecular weight of the liquid-phase material is higher than 6,000;
a third depolymerization reaction unit, wherein the liquid-phase material with a molecular weight higher than 6,000 from the second depolymerization reaction unit circulates in the third depolymerization reaction unit and carries out the reaction, until the molecular weight of the liquid-phase material is higher than 10,000; and
a device for collecting and purifying the gas-phase crude lactide from the first depolymerization reaction unit, the second depolymerization reaction unit, and the third depolymerization reaction unit.

Preferably, the first depolymerization reaction unit comprises a first depolymerization reactor and a first circulation tank, the lactic acid oligomer and the depolymerization catalyst carry out the reaction in the first depolymerization reactor, and the liquid-phase material obtained after the reaction enters the first circulation tank.

Preferably, the second depolymerization reaction unit comprises at least one second depolymerization reactor and at least one second circulation tank, the liquid-phase material derived from the first depolymerization reaction unit and an optional protonated solvent carry out the reaction in the second depolymerization reactor, the reacted liquid-phase material enters the second circulation tank; when the molecular weight of the liquid-phase material is 6,000 or less, the liquid-phase material in the second circulation tank is recycled to the second depolymerization reactor for further reaction; when the molecular weight of the liquid-phase material is larger than 6,000, the liquid-phase material in the second circulation tank is conveyed to the third depolymerization reaction unit for reaction.

Preferably, the third depolymerization reaction unit comprises at least one third depolymerization reactor and at least one third circulation tank, the liquid-phase material having a molecular weight larger than 6,000 derived from the second depolymerization reaction unit carries out the reaction in the third depolymerization reactor, the reacted liquid-phase material enters the third circulation tank; when the molecular weight of the liquid-phase material is 10,000 or less, the liquid-phase material in the third circulation tank is recycled to the third depolymerization reactor for further reaction; when the molecular weight of the liquid-phase material is larger than 10,000, the liquid-phase material in the third circulation tank is discharged out of the system.

In the system of the present invention, the reactors in the first depolymerization reaction unit, the second depolymerization reaction unit, and the third depolymerization reaction unit (i.e., the first depolymerization reactor, the second depolymerization reactor, and the third depolymerization reactor) are each a wiped film depolymerization reactor, preferably a thin film evaporator, a molecular distillation evaporator, or other stirred film evaporator.

According to a specific embodiment of the present invention, as shown in FIG. 1, the system for continuously preparing lactide by step control comprises: a first depolymerization reaction unit, a second depolymerization reaction unit, and a third depolymerization reaction unit; wherein the first depolymerization reaction unit comprises a first polymerization reactor I and a first circulation tank IV, the second depolymerization reaction unit comprises a second polymerization reactor II and a second circulation tank V, the second circulation tank V is configured with a first molecular weight detection and control assembly VII, the third depolymerization reaction unit comprises a third polymerization reactor III and a third circulation tank VI, the third circulation tank VI is configured with a second molecular weight detection and control assembly VIII. The specific operation process of the system is as follows: firstly, transporting lactic acid oligomer 01 to a first depolymerization reactor I for reaction, discharging the unreacted lactic acid oligomer into a first circulation tank IV, controlling the pressure to be within a range from 10 kPa to atmospheric pressure, controlling the temperature to be within the range of 160-200°C, when the liquid level in the tank reaches 50% -70%, transporting a first circulation tank discharge 05 or a mixture of the first circulation tank discharge 05 and protonated solvent 10 to a second depolymerization reactor II for reaction, conveying the unreacted lactic acid oligomer into a second circulation tank V, controlling the pressure to be within the range from 10 kPa to atmospheric pressure, controlling the temperature to be within the range of 160-200°C, when the liquid level in the tank reaches 50%-70%, measuring the molecular weight of the lactic acid oligomer in the second circulation tank V, if the molecular weight is not higher than 6,000 (preferably within a range of 3,000-6,000), using the lactic acid oligomer as a second circulation tank recycled material 06 and returning it to the second depolymerization reactor II; if the molecular weight is larger than 6,000, using the lactic acid oligomer as a second circulation tank discharge 07 and conveying it to the third depolymerization reactor III for reaction, the unreacted lactic acid oligomer enters the third circulation tank VI, controlling the pressure to be within the range from 10 kPa to atmospheric pressure, controlling the temperature to be within the range of 160-200°C, when the liquid level in the tank reaches 10% -30%, measuring the molecular weight of the oligomer in the third circulation tank VI, if the molecular weight is not higher than 10,000, using the lactic acid oligomer as a third circulation tank recycled material 08 and returning it to the third depolymerization reactor III; if the molecular weight is larger than 10,000, using the lactic acid oligomer as a lactic acid high polymer 09 and discharging it out of the system, the discharged material is lactic acid high polymer, which can be hydrolyzed into lactic acid for recycling. The gas-phase crude lactide generated from the three depolymerization reactors is discharged from the top of each depolymerization reactor to obtain a crude lactide product 04, which enters a separation and purification process.

The method and system for preparing lactide of the present invention will be further described with reference to examples. The examples are implemented on the premise of the technical scheme of the invention, and give the detailed embodiments and specific operation processes, but the protection scope of the invention is not limited to the following examples.

Unless otherwise specified, the experimental methods in the following examples were the conventional methods in the art. Unless otherwise indicated, the experimental materials used in the following examples were commercially available from the biochemical reagent stores in China.

The lactic acid adopted by the examples of the invention was the heat-resistant L-lactic acid having a lactic acid content of 88% or above, and an optical purity of 99.0% or higher.

The molecular weight of the lactic acid oligomer was measured by using the Viscotek OMNISEC GPC/SEC gel permeation chromatograph Manufactured by Malvern Instruments Limited in the United Kingdom (UK). The traditional calibration method was adopted, Polystyrene (PS) was taken as an internal standard, the model of a chromatographic column was T3000, the dimensions were 300mmL×8.0mm, the column temperature was 40°C, the flow rate was 1.0mL/min, the sample concentration was within a range of 2-5mg/mL, and the single sample feed amount was 500µL.

The invention adopted the Agilent High-Performance Liquid Chromatograph (HPLC) to analyze the chemical purity of lactide, the contents of L-lactic acid, dimer and trimer; an ultraviolet detector used phosphoric acid and acetonitrile as mobile phases, the chromatographic column model was ZORBAX SB-Aq, the column length was 250mm, a column inner diameter was 4.6mm, and the column was filled with a filler having a particle size of 5µm. Detection wavelength: 200nm, column temperature: 40°C, flow rate: 1mL/min, sample feed amount: 5µL.

The invention used the Agilent gas chromatograph to analyze the lactide contents of different optical isomers, selected a CYCLOSIL-B type chromatographic column, the temperature of a gasification chamber was 250°C, the temperature of a detector was 280°C and adopted a hydrogen flame ion detector, the column temperature was subjected to the programmed temperature rise, wherein the initial temperature was 100°C, the temperature was kept for 5min, and then heated to 140°C at a temperature rise rate of 4°C/min, the temperature was kept at 140°C for 7 min, and then increased to 200°C at a temperature rise rate of 8°C/min, the temperature was maintained for 20min, the flow rate of carrier gas N₂ was 1.4mL/min, the flow rate of hydrogen gas was 30mL/min, the flow rate of air was 400mL/min, and the sample feed amount was 0.5µL.

The yield *Y* of the lactide during the purification process and the product yield Yₜₒₜₐₗ during the whole preparation and purification process were calculated according to the following formula:
$Y = m / \left({m}_{0}\times {y}_{0}\right) \times 100 %$
${Y}_{total} = m / M \times 100 %$

Wherein m₀ denoted the mass of the crude lactide, y₀ denoted the purity of L-lactide in the crude lactide, m denoted the mass of a lactide product, and M denoted the mass of lactide which can be theoretically converted from a certain amount of lactic acid oligomer (i.e., the mass of lactic acid oligomer).

The specific rotation of the sample was analyzed by adopting a WZZ-2S automatic polarimeter, to represent the optical purity of the sample, wherein the specific rotation of pure L-lactide was -278, the specific rotation of pure D-lactide was +278, the specific rotation of m-lactide was 0, and the calculation formula of the optical purity X of the sample was as follows; ${X}_{optical purity} = \left[1-\frac{\left|{α}_{pure substance}-{α}_{tested sample}\right|}{{α}_{pure substance}}\right] \times 100 %$
wherein the α_{pure substance} denoted the specific rotation of pure lactide, and the α_{tested sample} represents the specific rotation of the tested substance.

The examples of the invention were carried out according to the device and process shown in FIG. 1, firstly, the lactic acid oligomer 01 was transported to the first scraped film depolymerization reactor I for reaction, the unreacted lactic acid oligomer was discharged into the circulation tank IV, the pressure was controlled to be within a range from 10 kPa to atmospheric pressure, the temperature was controlled to be within the range of 160-200°C, when the liquid level in the tank reached 50% -70%, a first circulation tank discharge 05 or a mixture of the first circulation tank discharge 05 and protonated solvent 10 was conveyed to a second wiped film depolymerization reactor II for reaction, the unreacted lactic acid oligomer entered a second circulation tank V, the pressure was controlled to be within the range from 10 kPa to atmospheric pressure, the temperature was controlled to be within the range of 160-200°C, when the liquid level in the tank reached 50% -70%, the molecular weight of the lactic acid oligomer in the second circulation tank V was measured, if the molecular weight was not higher than 6,000 (preferably within a range of 3,000-6,000), the lactic acid oligomer was used as a second circulation tank recycled material 06 and returned to the second depolymerization reactor II; if the molecular weight was larger than 6,000, the lactic acid oligomer was used as a second circulation tank discharge 07 and conveyed to the third wiped film depolymerization reactor III for reaction, the unreacted lactic acid oligomer entered the third circulation tank VI, the pressure was controlled to be within the range from 10 kPa to atmospheric pressure, the temperature was controlled to be within the range of 160-200°C, when the liquid level in the tank reached 10% - 30%, the molecular weight of the oligomer in the third circulation tank VI was measured, if the molecular weight was not higher than 10,000, the lactic acid oligomer was used as a third circulation tank recycled material 08 and returned to the third depolymerization reactor III; if the molecular weight was larger than 10,000, the lactic acid oligomer was used as a lactic acid high polymer 09 and discharged out of the system, the discharged material was lactic acid high polymer 10, which can be hydrolyzed into lactic acid for recycling. The gas-phase crude lactide generated from the three depolymerization reactors was discharged from the top of each depolymerization reactor, and a crude lactide product 04 was obtained, which entered a separation and purification process.

Preparation of lactic acid oligomer:
(1) Removal of free water from the lactic acid: 4,000g L-lactic acid (wherein the content of lactic acid was about 88.0%, the optical purity was 99.2%) was weighted and added into a reaction kettle with a stirring system, a vacuum circulating water pump was used for maintaining the pressure of the system at about 50kPa, the lactic acid was heated heating under vacuum environment, the temperature was gradually raised to 110-120°C, the dehydration was carried out for 2h, the free water in the reaction system was slowly evaporated out of the reaction system during the dehydration process.
(2) Preparation of lactic acid oligomer: after the free water in the system was almost completely removed, the vacuum degree of the system was increased, the pressure of the system was slowly reduced to about 1.2kPa, the temperature of the feed liquid was gradually increased to 160°C, reaction was performed for 2.5h, in the meanwhile, the polycondensation reaction among the lactic acid molecules was carried out, and the moisture generated in the reaction in the system was evaporated out of the system, the lactic acid oligomer with a molecular weight of 1,901 was obtained.

### Example 1

3,000g of lactic acid oligomer was weighted, 30g of stannous octoate catalyst was added, the materials were uniformly blended and conveyed to a first wiped film depolymerization reactor, the depolymerization reaction conditions were controlled as follows: the vacuum degree was 600Pa, the reaction temperature was 190°C, the one-way reaction time was 4min, the unreacted lactic acid oligomer was discharged into a first circulation tank, the temperature was controlled at 180°C, the pressure was controlled at 20kPa, after the liquid level was increased to 60%, the molecular weight of lactic acid oligomer was measured to be 3,216. The conversion rate of lactic acid oligomer during the process was 54.6%.

The lactic acid oligomer in the first circulation tank was conveyed to a second wiped film depolymerizing reactor, the reaction temperature was controlled to be 210°C, the vacuum degree was controlled to be 400Pa, the one-way reaction time was controlled to be 3min, the feeding amount of the lactic acid oligomer was 4 times of the actual reaction amount, the unreacted lactic acid oligomer was discharged into a second circulation tank, the liquid level was maintained at 50%, the pressure was kept at 10kPa, and the temperature was maintained at 180°C, the molecular weight of the lactic acid oligomer in the second circulation tank was measured to be 4,915, the lactic acid oligomer was controlled and recycled to the second depolymerization reactor for continuously participating in the reaction; when the molecular weight of oligomer at the second circulation tank outlet was measured to be larger than 6,000, the oligomer was conveyed to a third depolymerization reactor. The conversion rate of lactic acid oligomer during the process was 72.4%.

The lactic acid oligomer in the second circulation reactor was conveyed to a third depolymerization reactor, wherein the reaction temperature was 230°C, the vacuum degree was 300Pa, the one-way reaction time was 2min, the feeding amount of the lactic acid oligomer was 5 times of the actual reaction amount, the unreacted lactic acid oligomer was discharged into the third circulation tank, the temperature was controlled at 180°C, the pressure was controlled at 20kPa; after the liquid level was increased to 20%, the molecular weight of the lactic acid oligomer was measured to be 7,864, the lactic acid oligomer was recycled to the third depolymerization reactor for continuously participating in the reaction; after the molecular weight of the lactic acid oligomer was increased to 10,000, the lactic acid oligomer was discharged out of the system. The conversion rate of lactic acid oligomer during the process was 71.1%.

The crude lactide obtained from the whole process was composed of the following ingredients: 89.7% of L-lactide, 3.2% of m-lactide, 2.5% of L-lactic acid, and 3.4% of lactic acid dimer and trimer. The conversion rate of the lactic acid oligomer in the whole cascade cyclic depolymerization process can reach 98.3%.

The crude lactide product was purified and refined through the two-stage rectification system, both the chemical purity and the optical purity of the purified and refined product can meet the requirements of polymer-grade lactide monomers.

### Example 2

3,000g of lactic acid oligomer was weighted, 30g of stannous octoate catalyst was added, the materials were uniformly blended and conveyed to a first wiped film depolymerization reactor, the depolymerization reaction conditions were controlled as follows: the vacuum degree was 1,500Pa, the reaction temperature was 230°C, the one-way reaction time was 8min, the unreacted lactic acid oligomer was discharged into a first circulation tank, the temperature was controlled at 200°C, the pressure was controlled at 20kPa; after the liquid level was increased to 60%, the molecular weight of lactic acid oligomer was measured to be 3,713. The conversion rate of lactic acid oligomer during the process was 50.8%.

The lactic acid oligomer in the first circulation tank was conveyed to a second wiped film depolymerizing reactor, the reaction temperature was controlled to be 220°C, the vacuum degree was controlled to be 1,000Pa, the one-way reaction time was controlled to be 5min, the feeding amount of the lactic acid oligomer was 3 times of the actual reaction amount, the unreacted lactic acid oligomer was discharged into a second circulation tank, the liquid level was maintained at 50%, the pressure was kept at 20kPa, and the temperature was maintained at 180°C, the molecular weight of the lactic acid oligomer in the second circulation tank was measured to be 6,032, the lactic acid oligomer was conveyed to the third depolymerization reactor. The conversion rate of lactic acid oligomer during the process was 71.2%.

The lactic acid oligomer in the second circulation reactor was conveyed to a third depolymerization reactor, wherein the reaction temperature was 240°C, the vacuum degree was 800Pa, the one-way reaction time was 4min, the feeding amount of the lactic acid oligomer was 4 times of the actual reaction amount, the unreacted lactic acid oligomer was discharged into the third circulation tank, the temperature was controlled at 180°C, the pressure was controlled at 20kPa; after the liquid level was increased to 20%, the molecular weight of the lactic acid oligomer was measured to be 7,357, the lactic acid oligomer was recycled to the third depolymerization reactor for continuously participating in the reaction; after the molecular weight of the lactic acid oligomer was increased to 10,000, the lactic acid oligomer was discharged out of the system. The conversion rate of lactic acid oligomer during the process was 70.3%.

The crude lactide obtained from the whole process was composed of the following ingredients: 84.6% of L-lactide, 5.9% of m-lactide, 1.5% of L-lactic acid, and 4.0% of lactic acid dimer and trimer. The conversion rate of the lactic acid oligomer in the whole cascade cyclic depolymerization process can reach 97.1%.

The crude lactide product was purified and refined through the two-stage rectification system, both the chemical purity and the optical purity of the purified and refined product can meet the requirements of polymer-grade lactide monomers.

### Example 3

3,000g of lactic acid oligomer was weighted, 30g of stannous octoate catalyst was added, the materials were uniformly blended and conveyed to a first wiped film depolymerization reactor, the depolymerization reaction conditions were controlled as follows: the vacuum degree was 500Pa, the reaction temperature was 180°C, the one-way reaction time was 3min, the feeding amount of the lactic acid oligomer was 4 times of the actual reaction amount, the unreacted lactic acid oligomer was discharged into a first circulation tank, the temperature was controlled at 180°C, the pressure was controlled at 20kPa, after the liquid level was increased to 60%, the molecular weight of lactic acid oligomer was measured to be 3,461. The conversion rate of lactic acid oligomer during the process was 51.8%.

The lactic acid oligomer in the first circulation tank was conveyed to a second wiped film depolymerizing reactor, the reaction temperature was controlled to be 200°C, the vacuum degree was controlled to be 400Pa, the one-way reaction time was controlled to be 2min, the feeding amount of the lactic acid oligomer was 5 times of the actual reaction amount, the unreacted lactic acid oligomer was discharged into a second circulation tank, the liquid level was maintained at 50%, the pressure was kept at 20kPa, and the temperature was maintained at 180°C, the molecular weight of the lactic acid oligomer in the second circulation tank was measured to be 4,456, the lactic acid oligomer was controlled and recycled to the second depolymerization reactor for continuously participating in the reaction; when the molecular weight of oligomer at the second circulation tank outlet was measured to be larger than 6,000, the oligomer was conveyed to a third depolymerization reactor. The conversion rate of lactic acid oligomer during the process was 71.9%.

The lactic acid oligomer in the second circulation reactor was conveyed to a third depolymerization reactor, wherein the reaction temperature was 220°C, the vacuum degree was 200Pa, the one-way reaction time was 1min, the feeding amount of the lactic acid oligomer was 6 times of the actual reaction amount, the unreacted lactic acid oligomer was discharged into the third circulation tank, the temperature was controlled at 180°C, the pressure was controlled at 20kPa; after the liquid level was increased to 20%, the molecular weight of the lactic acid oligomer was measured to be 7,071, the lactic acid oligomer was recycled to the third depolymerization reactor for continuously participating in the reaction; after the molecular weight of the lactic acid oligomer was increased to 10,000, the lactic acid oligomer was discharged out of the system. The conversion rate of lactic acid oligomer during the process was 72.4%.

The crude lactide obtained from the whole process was composed of the following ingredients: 89.2% of L-lactide, 3.2% of m-lactide, 2.9% of L-lactic acid, and 4.7% of lactic acid dimer and trimer. The conversion rate of the lactic acid oligomer in the whole cascade cyclic depolymerization process can reach 97.6%.

The crude lactide product was purified and refined through the two-stage rectification system, both the chemical purity and the optical purity of the purified and refined product can meet the requirements of polymer-grade lactide monomers.

### Example 4

Lactide was prepared according to the method of Example 1, except that the same mass of stannous chloride catalyst was used. After the reaction in the first depolymerization reactor, the molecular weight of lactic acid oligomer at the first circulation tank outlet was measured to be 3,527, and the conversion rate of lactic acid oligomer during the process was 53.8%. After the lactic acid oligomer entered the second depolymerization reactor for reaction, the molecular weight of the lactic acid oligomer in the second circulation tank was measured to be 5,319, the lactic acid oligomer was directly recycled to the second depolymerization reactor for continuously participating in the reaction; when the molecular weight of oligomer at the second circulation tank outlet was measured to be larger than 6,000, the oligomer was conveyed to a third depolymerization reactor, the conversion rate of lactic acid oligomer during the process was 71.7%. After the reaction in the third depolymerization reactor, the molecular weight of the lactic acid oligomer at the third depolymerization reactor outlet was measured to be 8,213, the lactic acid oligomer was recycled to the third depolymerization reactor for continuously participating in the reaction, after the molecular weight of the lactic acid oligomer was increased to 10,000, the lactic acid oligomer was discharged out of the system. The conversion rate of lactic acid oligomer during the process was 70.8%.

The crude lactide obtained from the whole process was composed of the following ingredients: 88.2% of L-lactide, 3.9% of m-lactide, 2.7% of L-lactic acid, and 4.4% of lactic acid dimer and trimer. The conversion rate of the lactic acid oligomer in the whole cascade cyclic depolymerization process can reach 97.8%.

The crude lactide product was purified and refined through the two-stage rectification system, both the chemical purity and the optical purity of the purified and refined product can meet the requirements of polymer-grade lactide monomers.

### Example 5

Lactide was prepared according to the method of Example 1, except that a depolymerization evaporator in the form of a molecular distillation was used. After the reaction in the first depolymerization reactor, the molecular weight of lactic acid oligomer at the first circulation tank outlet was measured to be 3,009, and the conversion rate of lactic acid oligomer during the process was 55.3%. After the lactic acid oligomer entered the second depolymerization reactor for reaction, the conversion rate of lactic acid oligomer during the process was 73.5%. After the reaction in the third depolymerization reactor, the conversion rate of lactic acid oligomer during the process was 72.7%.

The crude lactide obtained from the whole process was composed of the following ingredients: 91.3% of L-lactide, 2.2% of m-lactide, 2.9% of L-lactic acid, and 3.0% of lactic acid dimer and trimer. The conversion rate of the lactic acid oligomer in the whole cascade cyclic depolymerization process can reach 98.7%.

The crude lactide product was purified and refined through the two-stage rectification system, both the chemical purity and the optical purity of the purified and refined product can meet the requirements of polymer-grade lactide monomers.

### Example 6

Lactide was prepared according to the method of Example 1, except that 1.0% tetradecanediol was added before the unreacted lactic acid oligomers in the first depolymerization reactor were conveyed into the second depolymerization reactor. The conversion rate of lactic acid oligomer in the second depolymerization reactor was 74.4%. The conversion rate of lactic acid oligomer in the third depolymerization reactor was 72.4%.

The crude lactide obtained from the whole process was composed of the following ingredients: 91.3% of L-lactide, 1.8% of m-lactide, 2.1% of L-lactic acid, and 3.3% of lactic acid dimer and trimer. The conversion rate of the lactic acid oligomer in the whole cascade cyclic depolymerization process can reach 98.6%. The racemization degree of the crude lactide product obtained in each step was low, and the processing amount of materials in the whole process was high.

The crude lactide product was purified and refined through the two-stage rectification system, both the chemical purity and the optical purity of the purified and refined product can meet the requirements of polymer-grade lactide monomers.

### Example 7

Lactide was prepared according to the method of Example 1, except that 1.0% dodecanediamine was added before the unreacted lactic acid oligomers in the first depolymerization reactor were conveyed into the second depolymerization reactor. The conversion rate of lactic acid oligomer in the second depolymerization reactor was 72.9%. The conversion rate of lactic acid oligomer in the third depolymerization reactor was 71.9%.

The crude lactide obtained from the whole process was composed of the following ingredients: 91.5% of L-lactide, 1.6% of m-lactide, 2.2% of L-lactic acid, and 3.1% of lactic acid dimer and trimer. The conversion rate of the lactic acid oligomer in the whole cascade cyclic depolymerization process can reach 98.7%.

The crude lactide product was purified and refined through the two-stage rectification system, both the chemical purity and the optical purity of the purified and refined product can meet the requirements of polymer-grade lactide monomers.

### Comparative Example 1

Lactide was prepared according to the method of Example 1, except that the molecular weight of lactic acid oligomer in the circulation tanks was not measured and the regulation and control were not performed according to the measurement result. The crude lactide obtained from the whole process was composed of the following ingredients: 85.4% of L-lactide, 6.5% of m-lactide, 4.7% of L-lactic acid, and 5.8% of lactic acid dimer and trimer. The conversion rate of the lactic acid oligomer in the whole cascade cyclic depolymerization process can reach 95.3%. Although the quality of the obtained crude lactide product was equivalent to that of the product derived from step control, the conversion rate of lactic acid oligomer was lower, the reaction treatment capacity in the whole process was only about 200g/h (the treatment capacity can reach more than 300g/h in Example 1); in addition, along with the continuous increase of the molecular weight of the circulating oligomer, and the continuous change of the composition at the depolymerization reactor inlet, the depolymerization reaction rate was continuously changed, the racemization degree was gradually increased, the composition of an outlet product was in dynamic change, the reaction stability in the continuous process was poor, and the device was required to be stopped periodically for performing the deslagging operation.

### Comparative Example 2

The lactide was prepared by adopting the technical scheme of depolymerizing lactic acid oligomer in the patent application CN111153886A filed by the Nanjing University, namely, a first-stage cyclic depolymerization process was adopted; the form of a depolymerization reactor in the Comparative Example was the same as that in Example 1, and the depolymerization reaction conditions were controlled as follows: the vacuum degree was 300Pa, the reaction temperature was 210°C, the one-way retention time was about 2min, the heavy components after reaction were discharged into a circulation tank, and was mixed with fresh lactic acid oligomer and then conveyed into a depolymerization reactor, the mass ratio of fresh materials to circulating materials in the reaction process was controlled to be 1: 3; when the reaction proceeded, the liquid level in the circulation tank was controlled to be maintained at 60%, the pressure was kept at 50kPa, and the temperature was maintained at 180°C. The periodic deslagging was performed with reference to the molecular weight of lactic acid oligomer at the circulation tank outlet or the circulating cumulant of the catalyst, and the deslagging was carried out when the oligomer molecular weight was more than 10,000.

According to the analysis result, the crude lactide product was composed of the following ingredients: 85.2% of L-lactide, 8.6% of m-lactide, 2.4% of L-lactic acid, and 3.2% of lactic acid dimer and trimer. The conversion rate of the lactic acid oligomer in the synthesis process of crude lactide can reach 95.2%. Although the product yield can reach a high level of 95%, the product racemization was serious, and along with the reaction process, the material composition at the depolymerization reactor inlet changed greatly, so that the depolymerization reaction rate also fluctuated significantly, the depolymerization reaction time of the system was long, and the outlet material composition of the product was unstable.

### Comparative Example 3

The experiment of preparing lactide by depolymerizing lactic acid oligomer was carried out using a similar technical scheme with the Japanese patent application JPH08333359A, namely, the depolymerization was performed by using a three-reactor cascade, the material of the third reactor was recycled to the first reactor for reaction, the periodical deslagging from the bottom of the third reactor was implemented, and the depolymerization reactor was used in the same manner as that in Example 1. The three reactors connected in series had the same reaction temperature of 200°C, the vacuum degrees were sequentially increased and respectively 600Pa, 400Pa, and 300Pa (the same as those in Example 1); the feeding rate of the first depolymerization reactor was the same as that in Example 1, the conversion rate of the lactic acid oligomer in the first depolymerization reactor was 54.9%, the unreacted material in the first depolymerization reactor was continuously conveyed to the second depolymerization reactor, the conversion rate was 49.3%, the unreacted material of the second depolymerization reactor was conveyed to the third depolymerization reactor for reaction, the conversion rate was 40.1%, if the unreacted component was subjected to deslagging treatment in the meanwhile, the conversion rate of lactic acid oligomer in the whole reaction was 86%, the *m*-lactic acid content in the crude lactide product was 5.8%, but the composition of the lactic acid oligomer was relatively stable; however, if the unreacted components were recycled to the first depolymerization reactor in the meanwhile, the material composition at each reactor inlet would change greatly, although the product yield was improved, both the reaction efficiency and the stability of the reaction system were damaged.

As can be seen from the Examples and Comparative Examples mentioned above, the present invention adopts the multi-stage cascade depolymerization reaction and the step control, and performs regulation and control according to the molecular weight of lactic acid oligomer in the circulation tanks, thereby realizing the high-efficiency polymerization of lactic acid oligomer, reducing the racemization degree of lactide and the coking carbonization probability of the substrate, and ensuring the conversion rate of the lactic acid oligomer and the system stability throughout the whole reaction process. The conversion rate of lactic acid oligomer in the whole process may reach 97.0% or more.

The above content describes in detail the preferred embodiments of the present invention, the scope of the present invention is defined by the Claims.

## Claims

1. A method for continuously preparing lactide by step control, the method comprises the following steps:
(1) reacting a lactic acid oligomer and a depolymerization catalyst in a first depolymerization reaction unit to obtain a first liquid-phase material;
(2) circulating the first liquid-phase material in a second depolymerization reaction unit for reaction until the molecular weight of the liquid-phase material is higher than 6,000 to obtain a second liquid-phase material;
(3) circulating the second liquid-phase material in a third depolymerization reaction unit for reaction until the molecular weight of the liquid-phase material is higher than 10,000;
(4) collecting gas-phase crude lactide from the first depolymerization reaction unit, the second depolymerization reaction unit, and the third depolymerization reaction unit, and then purifying the same;
the second depolymerization reaction unit comprises at least one second depolymerization reactor and at least one second circulation tank, the first liquid-phase material and an optional protonated solvent carry out the reaction in the second depolymerization reactor, the reacted liquid-phase material enters the second circulation tank; when the molecular weight of the liquid-phase material is 6,000 or less, the liquid-phase material in the second circulation tank is recycled to the second depolymerization reactor for further reaction; when the molecular weight of the liquid-phase material is larger than 6,000, the liquid-phase material in the second circulation tank is conveyed to the third depolymerization reaction unit for reaction;
the third depolymerization reaction unit comprises at least one third depolymerization reactor and at least one third circulation tank, the second liquid-phase material carries out the reaction in the third depolymerization reactor, the reacted liquid-phase material enters the third circulation tank; when the molecular weight of the liquid-phase material is 10,000 or less, the liquid-phase material in the third circulation tank is recycled to the third depolymerization reactor for further reaction; when the molecular weight of the liquid-phase material is larger than 10,000, the liquid-phase material in the third circulation tank is discharged.

2. The method according to claim 1, wherein the lactic acid oligomer in step (1) has a molecular weight within a range of 800-3,000, preferably within a range of 1,200-2,800.

3. The method according to claim 1, wherein the polymerization catalyst in step (1) is used in an amount of 0.4%-3%, preferably 0.8%-2% by mass of the lactic acid oligomer;
preferably, the polymerization catalyst in step (1) is a tin-based catalyst, preferably at least one of stannous octoate, SnCl₂, and SnO.

4. The method according to claim 1, wherein the first depolymerization reaction unit comprises a first depolymerization reactor and a first circulation tank, the lactic acid oligomer and the depolymerization catalyst carry out the reaction in the first depolymerization reactor, and the first liquid-phase material obtained after the reaction enters the first circulation tank.

5. The method according to claim 1 or 4, wherein the conversion rate of the lactic acid oligomer in the reaction of step (1) is controlled to be between 50% and 60%.

6. The method according to claim 1, wherein the reaction of step (2) is performed in the presence of a protonated solvent, the protonated solvent is at least one of a diamine having 12 or more carbon atoms, and a diol having 12 or more carbon atoms;
preferably, the protonated solvent is at least one of C12-C18 diamine and C12-C18 diol, preferably at least one of dodecanediamine, tetradecanediamine, hexadecanediamine, tetradecanediol, and hexadecanediol.

7. The method according to claim 6, wherein the protonated solvent is used in an amount of 0.1%-6%, preferably 1%-3% by mass of the lactic acid oligomer in the reaction of step (2).

8. The method according to claim 1, when the molecular weight of the liquid-phase material is within a range of 3,000-6,000, the liquid-phase material in the second circulation tank is recycled to the second depolymerization reactor for further reaction; when the molecular weight of the liquid-phase material is more than 6,000 and less than 10,000, the liquid-phase material in the second circulation tank is conveyed to the third depolymerization reaction unit for reaction.

9. The method according to claim 1, wherein the feeding amount of the lactic acid oligomer in a one-way reaction of the second depolymerization reactor is 3-5 times the actual reaction amount.

10. The method according to claim 1, wherein the feeding amount of the lactic acid oligomer in the one-way reaction of the third depolymerization reactor is 4-6 times the actual reaction amount.

11. The method according to claim 1 or 4, wherein the reactors in the first depolymerization reaction unit, the second depolymerization reaction unit, and the third depolymerization reaction unit are each a wiped film depolymerization reactor, preferably a thin film evaporator, a molecular distillation evaporator, or other stirred film evaporator.

12. A system for continuously preparing lactide by step control, the system comprises:
a first depolymerization reaction unit, wherein a lactic acid oligomer and a polymerization catalyst carry out the reaction in the first depolymerization reaction unit;
a second depolymerization reaction unit, wherein a liquid-phase material from the first depolymerization reaction unit and an optional protonated solvent circulate in the second depolymerization reaction unit and carry out the reaction, until the molecular weight of the liquid-phase material is higher than 6,000;
a third depolymerization reaction unit, wherein the liquid-phase material with a molecular weight higher than 6,000 from the second depolymerization reaction unit circulates in the third depolymerization reaction unit and carries out the reaction, until the molecular weight of the liquid-phase material is higher than 10,000; and
a device for collecting and purifying the gas-phase crude lactide from the first depolymerization reaction unit, the second depolymerization reaction unit, and the third depolymerization reaction unit;
wherein the second depolymerization reaction unit comprises at least one second depolymerization reactor and at least one second circulation tank, the liquid-phase material derived from the first depolymerization reaction unit and an optional protonated solvent carry out the reaction in the second depolymerization reactor, the reacted liquid-phase material enters the second circulation tank; when the molecular weight of the liquid-phase material is 6,000 or less, the liquid-phase material in the second circulation tank is recycled to the second depolymerization reactor for further reaction;
when the molecular weight of the liquid-phase material is larger than 6,000, the liquid-phase material in the second circulation tank is conveyed to the third depolymerization reaction unit for reaction;
the third depolymerization reaction unit comprises at least one third depolymerization reactor and at least one third circulation tank, the liquid-phase material having a molecular weight larger than 6,000 derived from the second depolymerization reaction unit carries out the reaction in the third depolymerization reactor, the reacted liquid-phase material enters the third circulation tank; when the molecular weight of the liquid-phase material is 10,000 or less, the liquid-phase material in the third circulation tank is recycled to the third depolymerization reactor for further reaction;
when the molecular weight of the liquid-phase material is larger than 10,000, the liquid-phase material in the third circulation tank is discharged out of the system.

13. The system according to claim 12, wherein the first depolymerization reaction unit comprises a first depolymerization reactor and a first circulation tank, the lactic acid oligomer and the depolymerization catalyst carry out the reaction in the first depolymerization reactor, and the liquid-phase material obtained after the reaction enters the first circulation tank; and/or
the reactors in the first depolymerization reaction unit, the second depolymerization reaction unit, and the third depolymerization reaction unit are each a wiped film depolymerization reactor, preferably a thin film evaporator, a molecular distillation evaporator, or other stirred film evaporator.

## Patentansprüche

1. Verfahren zur kontinuierlichen Herstellung von Lactid durch Schrittsteuerung, wobei das Verfahren die folgenden Schritte umfasst:
(1) Umsetzen eines Milchsäureoligomers und eines Depolymerisationskatalysators in einer ersten Depolymerisationsreaktionseinheit, um ein erstes Flüssigphasenmaterial zu erhalten;
(2) Zirkulieren des ersten Flüssigphasenmaterials in einer zweiten Depolymerisationsreaktionseinheit zur Reaktion, bis das Molekulargewicht des Flüssigphasenmaterials über 6.000 liegt, um ein zweites Flüssigphasenmaterial zu erhalten;
(3) Zirkulieren des zweiten Flüssigphasenmaterials in einer dritten Depolymerisationsreaktionseinheit zur Reaktion, bis das Molekulargewicht des Flüssigphasenmaterials größer als 10.000 ist;
(4) Sammeln von rohem Lactid in der Gasphase aus der ersten Depolymerisationsreaktionseinheit, der zweiten Depolymerisationsreaktionseinheit und der dritten Depolymerisationsreaktionseinheit und anschließende Reinigung desselben;
die zweite Depolymerisationsreaktionseinheit umfasst mindestens einen zweiten Depolymerisationsreaktor und mindestens einen zweiten Zirkulationstank; das erste Flüssigphasenmaterial und ein optionales protoniertes Lösungsmittel durchlaufen die Reaktion im zweiten Depolymerisationsreaktor; das Flüssigphasenmaterial, das reagiert hat, gelangt in den zweiten Zirkulationstank; wenn das Molekulargewicht des Flüssigphasenmaterials 6.000 oder weniger beträgt, wird das Flüssigphasenmaterial im zweiten Zirkulationstank zur weiteren Reaktion in den zweiten Depolymerisationsreaktor zurückgeführt; wenn das Molekulargewicht des Flüssigphasenmaterials größer als 6.000 ist, wird das Flüssigphasenmaterial im zweiten Zirkulationstank zur Reaktion in die dritte Depolymerisationsreaktionseinheit geleitet;
die dritte Depolymerisationsreaktionseinheit umfasst mindestens einen dritten Depolymerisationsreaktor und mindestens einen dritten Zirkulationstank; das zweite Flüssigphasenmaterial durchläuft die Reaktion im dritten Depolymerisationsreaktor, und das Flüssigphasenmaterial, das reagiert hat, gelangt in den dritten Zirkulationstank; wenn das Molekulargewicht des Flüssigphasenmaterials 10.000 oder weniger beträgt, wird das Flüssigphasenmaterial im dritten Zirkulationstank zur weiteren Reaktion in den dritten Depolymerisationsreaktor zurückgeführt; wenn das Molekulargewicht des Flüssigphasenmaterials größer als 10.000 ist, wird das Flüssigphasenmaterial im dritten Zirkulationstank abgelassen.

2. Verfahren nach Anspruch 1, wobei das Milchsäureoligomer in Schritt (1) ein Molekulargewicht im Bereich von 800-3.000, vorzugsweise im Bereich von 1.200-2.800 aufweist.

3. Verfahren nach Anspruch 1, wobei der Polymerisationskatalysator in Schritt (1) in einer Menge von 0,4-3 %, vorzugsweise 0,8-2 %, bezogen auf die Masse des Milchsäureoligomers, eingesetzt wird;
vorzugsweise ist der Polymerisationskatalysator in Schritt (1) ein Katalysator auf Zinnbasis, vorzugsweise mindestens einer aus: Zinn(II)-octoat, SnCl₂ und SnO.

4. Verfahren nach Anspruch 1, wobei die erste Depolymerisationsreaktionseinheit einen ersten Depolymerisationsreaktor und einen ersten Zirkulationstank umfasst, das Milchsäureoligomer und der Depolymerisationskatalysator die Reaktion im ersten Depolymerisationsreaktor durchlaufen und das nach der Reaktion erhaltene erste Flüssigphasenmaterial in den ersten Zirkulationstank gelangt.

5. Verfahren nach Anspruch 1 oder 4, wobei der Umwandlungsgrad des Milchsäureoligomers bei der Reaktion in Schritt (1) so gesteuert wird, dass er zwischen 50 % und 60 % liegt.

6. Verfahren nach Anspruch 1, wobei die Reaktion in Schritt (2) in Gegenwart eines protonierten Lösungsmittels durchgeführt wird, wobei das protonierte Lösungsmittel mindestens eines ist aus: ein Diamin mit 12 oder mehr Kohlenstoffatomen, und ein Diol mit 12 oder mehr Kohlenstoffatomen;
vorzugsweise ist das protonierte Lösungsmittel mindestens eines aus: C12-C18-Diamin und ein C12-C18-Diol, vorzugsweise mindestens eines aus: Dodecandiamin, Tetradecandiamin, Hexadecandiamin, Tetradecandiol und Hexadecandiol.

7. Verfahren nach Anspruch 6, wobei das protonierte Lösungsmittel in einer Menge von 0,1 % bis 6 %, vorzugsweise 1 % bis 3 %, bezogen auf die Masse des Milchsäureoligomers, bei der Reaktion in Schritt (2) verwendet wird.

8. Verfahren nach Anspruch 1, wobei, wenn das Molekulargewicht des Flüssigphasenmaterials im Bereich von 3.000-6.000 liegt, das Flüssigphasenmaterial im zweiten Zirkulationstank zur weiteren Reaktion in den zweiten Depolymerisationsreaktor zurückgeführt wird; wenn das Molekulargewicht des Flüssigphasenmaterials mehr als 6.000 und weniger als 10.000 beträgt, wird das Flüssigphasenmaterial im zweiten Zirkulationstank zur Reaktion in die dritte Depolymerisationsreaktionseinheit geleitet.

9. Verfahren nach Anspruch 1, wobei die Zufuhrmenge des Milchsäureoligomers in einer Einwegreaktion des zweiten Depolymerisationsreaktors das 3- bis 5-fache der tatsächlichen Reaktionsmenge beträgt.

10. Verfahren nach Anspruch 1, wobei die Zufuhrmenge des Milchsäureoligomers in der Einwegreaktion des dritten Depolymerisationsreaktors das 4- bis 6-fache der tatsächlichen Reaktionsmenge beträgt.

11. Verfahren nach Anspruch 1 oder 4, wobei die Reaktoren in der ersten Depolymerisationsreaktionseinheit, der zweiten Depolymerisationsreaktionseinheit und der dritten Depolymerisationsreaktionseinheit jeweils ein Wiped-Film-Depolymerisationsreaktor, vorzugsweise ein Dünnschichtverdampfer, ein Molekulardestillationsverdampfer oder ein anderer Rührfilmverdampfer sind.

12. System zur kontinuierlichen Herstellung von Lactid durch Schrittsteuerung, wobei das System umfasst:
eine erste Depolymerisationsreaktionseinheit, in der ein Milchsäureoligomer und ein Polymerisationskatalysator die Reaktion in der ersten Depolymerisationsreaktionseinheit durchlaufen;
eine zweite Depolymerisationsreaktionseinheit, in der ein Flüssigphasenmaterial aus der ersten Depolymerisationsreaktionseinheit und optional ein protoniertes Lösungsmittel in der zweiten Depolymerisationsreaktionseinheit zirkulieren und die Reaktion durchlaufen, bis das Molekulargewicht des Flüssigphasenmaterials größer als 6.000 ist;
eine dritte Depolymerisationsreaktionseinheit, in der das Flüssigphasenmaterial mit einem Molekulargewicht von mehr als 6.000 aus der zweiten Depolymerisationsreaktionseinheit in der dritten Depolymerisationsreaktionseinheit zirkuliert und die Reaktion durchläuft, bis das Molekulargewicht des Flüssigphasenmaterials größer als 10.000 ist; sowie eine Vorrichtung zum Auffangen und Reinigen des in der Gasphase vorliegenden rohen Lactids aus der ersten Depolymerisationsreaktionseinheit, der zweiten Depolymerisationsreaktionseinheit und der dritten Depolymerisationsreaktionseinheit;
wobei die zweite Depolymerisationsreaktionseinheit mindestens einen zweiten Depolymerisationsreaktor und mindestens einen zweiten Zirkulationstank umfasst, das aus der ersten Depolymerisationsreaktionseinheit stammende Flüssigphasenmaterial und ein optionales protoniertes Lösungsmittel die Reaktion im zweiten Depolymerisationsreaktor durchlaufen und das Flüssigphasenmaterial, das reagiert hat, in den zweiten Zirkulationstank gelangt; wenn das Molekulargewicht des Flüssigphasenmaterials 6.000 oder weniger beträgt, wird das Flüssigphasenmaterial im zweiten Zirkulationstank zur weiteren Reaktion in den zweiten Depolymerisationsreaktor zurückgeführt; wenn das Molekulargewicht des Flüssigphasenmaterials größer als 6.000 ist, wird das Flüssigphasenmaterial im zweiten Zirkulationstank zur Reaktion in die dritte Depolymerisationsreaktionseinheit geleitet;
die dritte Depolymerisationsreaktionseinheit umfasst mindestens einen dritten Depolymerisationsreaktor und mindestens einen dritten Zirkulationstank; das aus der zweiten Depolymerisationsreaktionseinheit stammende Flüssigphasenmaterial mit einem Molekulargewicht von mehr als 6.000 durchläuft die Reaktion im dritten Depolymerisationsreaktor, und das Flüssigphasenmaterial, das reagiert hat, gelangt in den dritten Zirkulationstank; wenn das Molekulargewicht des Flüssigphasenmaterials 10.000 oder weniger beträgt, wird das Flüssigphasenmaterial im dritten Zirkulationstank zur weiteren Reaktion in den dritten Depolymerisationsreaktor zurückgeführt; wenn das Molekulargewicht des Flüssigphasenmaterials größer als 10.000 ist, wird das Flüssigphasenmaterial im dritten Zirkulationstank aus dem System abgeleitet.

13. System nach Anspruch 12, wobei die erste Depolymerisationsreaktionseinheit einen ersten Depolymerisationsreaktor und einen ersten Zirkulationstank umfasst, das Milchsäureoligomer und der Depolymerisationskatalysator die Reaktion im ersten Depolymerisationsreaktor durchlaufen und das nach der Reaktion erhaltene Flüssigphasenmaterial in den ersten Zirkulationstank gelangt; und/oder
wobei die Reaktoren in der ersten Depolymerisationsreaktionseinheit, der zweiten Depolymerisationsreaktionseinheit und der dritten Depolymerisationsreaktionseinheit jeweils ein Wiped-Film-Depolymerisationsreaktor, vorzugsweise ein Dünnschichtverdampfer, ein Molekulardestillationsverdampfer oder ein anderer Rührfilmverdampfer sind.

## Revendications

1. Procédé de préparation en continu de lactide par commande d'étapes, ledit procédé comprenant les étapes suivantes :
(1) le fait de faire réagir un oligomère d'acide lactique et un catalyseur de dépolymérisation dans une première unité de réaction de dépolymérisation afin d'obtenir un premier matériau en phase liquide ;
(2) le fait de faire circuler le premier matériau en phase liquide dans une deuxième unité de réaction de dépolymérisation jusqu'à ce que le poids moléculaire du matériau en phase liquide soit supérieur à 6 000, afin d'obtenir un second matériau en phase liquide ;
(3) le fait de faire circuler le second matériau en phase liquide dans une troisième unité de réaction de dépolymérisation pour réaction jusqu'à ce que le poids moléculaire du matériau en phase liquide soit supérieur à 10 000 ;
(4) la collecte du lactide brut en phase gazeuse à partir de la première unité de réaction de dépolymérisation, de la deuxième unité de réaction de dépolymérisation et de la troisième unité de réaction de dépolymérisation, puis sa purification ;
la deuxième unité de réaction de dépolymérisation comprend au moins un deuxième réacteur de dépolymérisation et au moins un deuxième réservoir de circulation, le premier matériau en phase liquide et un éventuel solvant protoné effectuent la réaction dans le deuxième réacteur de dépolymérisation ; le matériau en phase liquide ayant réagi pénètre dans le deuxième réservoir de circulation ; lorsque le poids moléculaire du matériau en phase liquide est inférieur ou égal à 6 000, le matériau en phase liquide dans le deuxième réservoir de circulation est recyclé vers le deuxième réacteur de dépolymérisation pour une réaction supplémentaire ; lorsque le poids moléculaire du matériau en phase liquide est supérieur à 6 000, le matériau en phase liquide dans le deuxième réservoir de circulation est acheminé vers la troisième unité de réaction de dépolymérisation pour réaction ;
la troisième unité de réaction de dépolymérisation comprend au moins un troisième réacteur de dépolymérisation et au moins un troisième réservoir de circulation, le deuxième matériau en phase liquide effectue la réaction dans le troisième réacteur de dépolymérisation, le matériau en phase liquide ayant réagi pénètre dans le troisième réservoir de circulation ; lorsque le poids moléculaire du matériau en phase liquide est inférieur ou égal à 10 000, le matériau en phase liquide dans le troisième réservoir de circulation est recyclé vers le troisième réacteur de dépolymérisation pour une réaction supplémentaire ; lorsque le poids moléculaire du matériau en phase liquide est supérieur à 10 000, le matériau en phase liquide contenu dans le troisième réservoir de circulation est évacué.

2. Procédé selon la revendication 1, dans lequel l'oligomère d'acide lactique de l'étape (1) présente un poids moléculaire se situant dans une plage de 800 à 3 000, de préférence de 1 200 à 2 800.

3. Procédé selon la revendication 1, **caractérisé en ce que** le catalyseur de polymérisation de l'étape (1) est utilisé en une quantité de 0,4 % à 3 %, de préférence de 0,8 % à 2 %, en masse de l'oligomère d'acide lactique ;
de préférence, le catalyseur de polymérisation del'étape (1) est un catalyseur à base d'étain, de préférence au moins l'un parmi l'octoate stanneux, le SnCl₂ et le SnO.

4. Procédé selon la revendication 1, dans lequel la première unité de réaction de dépolymérisation comprend un premier réacteur de dépolymérisation et un premier réservoir de circulation, l'oligomère d'acide lactique et le catalyseur de dépolymérisation effectuent la réaction dans le premier réacteur de dépolymérisation, et le premier matériau en phase liquide obtenu après la réaction pénètre dans le premier réservoir de circulation.

5. Procédé selon la revendication 1 ou 4, dans lequel le taux de conversion de l'oligomère d'acide lactique dans la réaction de l'étape (1) est commandé de manière à se situer entre 50 % et 60 %.

6. Procédé selon la revendication 1, dans lequel la réaction de l'étape (2) est effectuée en présence d'un solvant protoné, le solvant protoné étant au moins l'un parmi une diamine comportant 12 atomes de carbone ou plus et un diol comportant 12 atomes de carbone ou plus ;
de préférence, le solvant protoné est au moins l'un parmi une diamine en C12-C18 et un diol en C12-C18, de préférence au moins l'un parmi la dodécanediamine, la tétradécanediamine, l'hexadécanediamine, le tétradécanediol et l'hexadécanediol.

7. Procédé selon la revendication 6, dans lequel le solvant protoné est utilisé en une quantité comprise de 0,1 % à 6 %, de préférence de 1 % à 3 %, en masse par rapport à l'oligomère d'acide lactique, dans la réaction de l'étape (2).

8. Procédé selon la revendication 1, dans lequel, lorsque le poids moléculaire du matériau en phase liquide se situe dans une plage de 3 000 à 6 000, le matériau en phase liquide présent dans le deuxième réservoir de circulation est recyclé vers le deuxième réacteur de dépolymérisation pour une réaction supplémentaire ; lorsque le poids moléculaire du matériau en phase liquide est supérieur à 6 000 et inférieur à 10 000, le matériau en phase liquide dans le deuxième réservoir de circulation est acheminé vers la troisième unité de réaction de dépolymérisation pour réaction.

9. Procédé selon la revendication 1, dans lequel la quantité d'alimentation en oligomère d'acide lactique dans une réaction unidirectionnelle du deuxième réacteur de dépolymérisation est de 3 à 5 fois la quantité réelle de réaction.

10. Procédé selon la revendication 1, dans lequel la quantité d'alimentation en oligomère d'acide lactique dans la réaction unidirectionnelle du troisième réacteur de dépolymérisation est de 4 à 6 fois la quantité réelle de réaction.

11. Procédé selon la revendication 1 ou 4, dans lequel les réacteurs de la première unité de réaction de dépolymérisation, de la deuxième unité de réaction de dépolymérisation et de la troisième unité de réaction de dépolymérisation sont chacun un réacteur de dépolymérisation à film raclé, de préférence un évaporateur à film mince, un évaporateur à distillation moléculaire ou un autre évaporateur à film agité.

12. Système pour la préparation en continu de lactide par commande d'étapes, le système comprenant :
une première unité de réaction de dépolymérisation, un oligomère d'acide lactique et un catalyseur de polymérisation effectuant la réaction dans la première unité de réaction de dépolymérisation ;
une deuxième unité de réaction de dépolymérisation, un matériau en phase liquide provenant de la première unité de réaction de dépolymérisation et un éventuel solvant protoné circulant dans la deuxième unité de réaction de dépolymérisation et effectuant la réaction, jusqu'à ce que le poids moléculaire du matériau en phase liquide soit supérieur à 6 000 ;
une troisième unité de réaction de dépolymérisation, le matériau en phase liquide, dont le poids moléculaire est supérieur à 6 000, provenant de la deuxième unité de réaction de dépolymérisation, circulant dans la troisième unité de réaction de dépolymérisation et effectuant la réaction jusqu'à ce que le poids moléculaire du matériau en phase liquide soit supérieur à 10 000 ; et
un dispositif pour collecter et purifier le lactide brut en phase gazeuse provenant de la première unité de réaction de dépolymérisation, de la deuxième unité de réaction de dépolymérisation et de la troisième unité de réaction de dépolymérisation ;
dans lequel la deuxième unité de réaction de dépolymérisation comprend au moins un deuxième réacteur de dépolymérisation et au moins un deuxième réservoir de circulation, le matériau en phase liquide issu de la première unité de réaction de dépolymérisation et un éventuel solvant protoné effectuent la réaction dans le deuxième réacteur de dépolymérisation ; le matériau en phase liquide ayant réagi pénètre dans le deuxième réservoir de circulation ; lorsque le poids moléculaire du matériau en phase liquide est inférieur ou égal à 6.000, le matériau en phase liquide dans le deuxième réservoir de circulation est recyclé vers le deuxième réacteur de dépolymérisation pour une réaction supplémentaire ; lorsque le poids moléculaire du matériau en phase liquide est supérieur à 6.000, le matériau en phase liquide dans le deuxième réservoir de circulation est acheminé vers la troisième unité de réaction de dépolymérisation pour réaction ;
la troisième unité de réaction de dépolymérisation comprend au moins un troisième réacteur de dépolymérisation et au moins un troisième réservoir de circulation, le matériau en phase liquide présentant un poid moléculaire supérieur à 6 000 issu de la deuxième unité de réaction de polymérisation effectue la réaction dans le troisième réacteur de dépolymérisation, le matériau en phase liquide ayant réagi pénètre dans le troisième réservoir de circulation ; lorsque le poids moléculaire du matériau en phase liquide est inférieur ou égal à 10.000, le matériau en phase liquide dans le troisième réservoir de circulation est recyclé vers le troisième réacteur de dépolymérisation pour une réaction supplémentaire ; lorsque le poids moléculaire du matériau en phase liquide est supérieur à 10.000, le matériau en phase liquide dans le troisième réservoir de circulation est évacué hors du système.

13. Système selon la revendication 12, dans lequel la première unité de réaction de dépolymérisation comprend un premier réacteur de dépolymérisation et un premier réservoir de circulation, l'oligomère d'acide lactique et le catalyseur de dépolymérisation effectuent la réaction dans le premier réacteur de dépolymérisation, et le premier matériau en phase liquide obtenu après la réaction pénètre dans le premier réservoir de circulation ; et/ou
les réacteurs de la première unité de réaction de dépolymérisation, de la deuxième unité de réaction de dépolymérisation et de la troisième unité de réaction de dépolymérisation sont chacun un réacteur de dépolymérisation à film raclé, de préférence un évaporateur à film mince, un évaporateur à distillation moléculaire ou un autre évaporateur à film agité.
